(19) Europäisches Patentamt
European Patent Office
Office européen des brevets

(11) EP 3 968 340 A1

(12) EUROPEAN PATENT APPLICATION
published in accordance with Art. 153(4) EPC

(43) Date of publication:
16.03.2022 Bulletin 2022/11

(51) Int Cl.:
*G16H 50/30* (2018.01)    *G16H 50/20* (2018.01)
*A61B 5/00* (2006.01)

(21) Application number: 19940912.9

(22) Date of filing: 13.08.2019

(86) International application number:
PCT/KR2019/010258

(87) International publication number:
WO 2021/025218 (11.02.2021 Gazette 2021/06)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 08.08.2019 KR 20190096589

(71) Applicant: Clinomics Inc.
Ulsan 44919 (KR)

(72) Inventors:
• **CHO, Yun Sung**
  **Yongin-Si Gyeonggi-do 16953 (KR)**
• **KIM, Byung Chul**
  **Suwon-Si Gyeonggi-do 16713 (KR)**
• **BHAK, Jong Hwa**
  **Ulju-Gun Ulsan 44936 (KR)**

(74) Representative: **Swindell & Pearson Limited**
**48 Friar Gate**
**Derby DE1 1GY (GB)**

(54) **DEVICE AND METHOD FOR PREDICTING RISK FOR DISEASE COMBINED WITH GENETIC RISK FOR ASSOCIATED PHENOTYPE**

(57) The present invention relates to a device and a method for predicting the risk for a disease combined with the genetic risk for an associated phenotype, in which a disease genetic risk for a disease and a phenotypic genetic risk for a phenotype (related diseases, lifestyle, etc.) related to the disease are obtained based on information on the genotype of a user, and a comprehensive genetic risk for the disease is obtained based on the disease genetic risk and the phenotypic genetic risk, and thus, more objective and accurate disease prediction is possible.

FIG. 2

```
            ┌─────────┐
            │  Start  │
            └────┬────┘
                 ▼
  ┌───────────────────────────────────────────┐
  │ Obtain disease genetic risk for disease on │
  │ basis of genetic marker information        │── S110
  │ associated with disease occurrence and     │
  │ information on genotype of user            │
  └───────────────────┬───────────────────────┘
                      ▼
  ┌───────────────────────────────────────────┐
  │ Obtain phenotype genetic risk for          │
  │ phenotype on basis of genetic marker       │── S130
  │ information on phenotype associated with   │
  │ disease occurrence and information on      │
  │ genotype of user                          │
  └───────────────────┬───────────────────────┘
                      ▼
  ┌───────────────────────────────────────────┐
  │ Obtain comprehensive genetic risk for      │
  │ disease on basis of obtained disease       │── S150
  │ genetic risk and obtained phenotypic       │
  │ genetic risk                              │
  └───────────────────┬───────────────────────┘
                      ▼
               ┌─────────┐
               │   End   │
               └─────────┘
```

**Description**

[Technical Field]

The present invention relates to a device and a method for predicting the risk for a disease combined with the genetic risk for an associated phenotype, and more particularly, to a device and a method for predicting the risk for a disease.

[Background Art]

Single nucleotide polymorphism (SNP) makers of genes related to various diseases or phenotypes have been found by genome sequence translation, genome-wide association study (GWAS), and the like.

Accordingly, it has been reported various methods for predicting the occurrence possibility of disease and non-disease phenotypes of a subject to be tested by using various genetic markers reported through existing technological studies (Reference: Schrodi SJ et al. Front. Genet. 2014), and even in domestic and overseas genetic testing agencies, the genetic risks for a disease and a phenotype have been tested by using similar methods.

However, except for the diseases (genetic diseases) that are 100% caused by genetic effects, the occurrence of most of general diseases (cancer, diabetes, etc.) is influenced even by the lifestyle or the presence or absence of other diseases in addition to genetic factors. Therefore, in order to accurately predict the occurrence possibility of diseases, in addition to the genetic factors, the comprehensive analysis of various lifestyles and environmental factors is also important. Recently, studies on disease prediction/diagnosis considering both genetic factors and non-genetic factors have been reported, and the Goldstein BA et al. Front. Genet. 2014 paper relates to a method for combing a clinical risk score and a genetic risk score for a coronary heart disease using a long-link function method. However, this method was a method which is applicable because a Framingham risk score (considering gender, age, cholesterol level, blood pressure, diabetes, smoking state, etc.) for clinically measuring/judging the risk for the coronary heart disease has been pre-established, and the values required for calculating the Framingham risk score have a disadvantage of requiring information measured by medical equipment such as health medical examination/medical examination.

In order to overcome the disadvantage, in Korean Patent Publication No. 2019-0077997, there is disclosed a method for predicting the occurrence possibility of a disease and a phenotype by collecting lifestyles and environmental factors affecting the disease occurrence through clinical questionnaire and combining the collected lifestyles and environmental factors with genetic factors.

However, in the case of collecting the phenotypes (lifestyle, environmental factors, and presence or absence of other related diseases) associated with the disease through the clinical questionnaire method, there are disadvantages that the subjects may answer the questionnaire items with different criteria depending on the subjective and judgment criteria of the subjects to be tested to cause inaccurate results, and the analysis is impossible when the subjects do not answer the clinical questionnaire. Therefore, it is necessary to develop a method of compensating for existing methods in predicting the disease by combining genetic factors and related phenotype factors for the disease.

[Disclosure]

[Technical Problem]

An object of the present invention is to provide a device and a method for predicting the risk for a disease combined with the genetic risk for an associated phenotype by obtaining a disease genetic risk for a disease and a phenotypic genetic risk for a phenotype (related diseases, lifestyle, etc.) associated with the disease based on information on a genotype of a user and obtaining a comprehensive genetic risk for the disease based on the disease genetic risk and the phenotypic genetic risk.

[Technical Solution]

One aspect of the present invention provides a device for predicting the risk of a disease combined with the genetic risk for an associated phenotype, the device including: a disease risk obtaining unit configured to obtain a disease genetic risk for the disease based on genetic marker information associated with the disease occurrence and information on a genotype of a user; a phenotypic risk obtaining unit configured to obtain a phenotypic genetic risk for a phenotype based on the genetic marker information on the phenotype associated with the disease occurrence and the information on the genotype of the user; and a comprehensive risk obtaining unit configured to obtain a comprehensive genetic risk for the disease based on the disease genetic risk obtained by the disease risk obtaining unit and the phenotypic genetic risk obtained by the phenotypic risk obtaining unit.

The comprehensive risk obtaining unit may obtain the comprehensive genetic risk based on the disease genetic risk and the phenotypic genetic risk by using a ratio of genetic factors affecting the disease occurrence.

The disease risk obtaining unit may convert the obtained disease genetic risk to a relative value as compared with disease genetic risks of other users in an affiliated group of the user, the phenotypic risk obtaining unit may convert the obtained phenotypic genetic risk to a relative value as compared with phenotypic genetic risks of other users in an affiliated group of the user, and the comprehensive risk obtaining unit may obtain the comprehensive genetic risk based on the disease genetic risk converted to the relative value and the phenotypic genetic risk converted to the relative value and convert the obtained comprehensive genetic risk to a relative value as compared with comprehensive genetic risks of other users in the affiliated group of the user.

The phenotypic risk obtaining unit may obtain the phenotypic genetic risk based on the genetic marker information on the phenotype and the information on the genotype of the user by using a predefined effective size by state of the phenotype.

The phenotypic risk obtaining unit may obtain a genetic risk based on the genetic marker information on the phenotype and the information on the genotype of the user, convert the obtained genetic risk to a relative value as compared with the genetic risks for the phenotypes of other users in the affiliated group of the user, obtain the state of the phenotype based on the genetic risk converted to the relative value, and determine an effective size corresponding to the obtained state as the phenotypic genetic risk.

When there is a plurality of phenotypes associated with the disease occurrence, the phenotypic risk obtaining unit may obtain a genetic risk for each of the plurality of phenotypes and may obtain a phenotypic genetic risk based on the obtained plurality of genetic risks.

Another aspect of the present invention provides a method for predicting the risk of a disease combined with the genetic risk for an associated phenotype, the method including: obtaining a disease genetic risk for the disease based on genetic marker information associated with the disease occurrence and information on a genotype of a user; obtaining a phenotypic genetic risk for a phenotype based on the genetic marker information on the phenotype associated with the disease occurrence and the information on the genotype of the user; and obtaining a comprehensive genetic risk for the disease based on the obtained disease genetic risk and the obtained phenotypic genetic risk.

The obtaining of the comprehensive genetic risk may be performed by obtaining the comprehensive genetic risk based on the disease genetic risk and the phenotypic genetic risk by using a ratio of genetic factors affecting the disease occurrence.

The obtaining of the disease genetic risk may be performed by converting the obtained disease genetic risk to a relative value as compared with disease genetic risks of other users in an affiliated group of the user, the obtaining of the phenotypic genetic risk may be performed by converting the obtained phenotypic genetic risk to a relative value as compared with phenotypic genetic risks of other users in an affiliated group of the user, and the obtaining of the comprehensive genetic risk may be performed by obtaining the comprehensive genetic risk based on the disease genetic risk converted to the relative value and the phenotypic genetic risk converted to the relative value and converting the obtained comprehensive genetic risk to a relative value as compared with comprehensive genetic risks of other users in the affiliated group of the user.

The obtaining of the phenotypic genetic risk may be performed by obtaining the phenotypic genetic risk based on the genetic marker information on the phenotype and the information on the genotype of the user by using a predefined effective size by state of the phenotype.

The obtaining of the phenotypic genetic risk may be performed by obtaining a genetic risk based on the genetic marker information on the phenotype and the information on the genotype of the user, converting the obtained genetic risk to a relative value as compared with the genetic risks for the phenotypes of other users in the affiliated group of the user, obtaining the state of the phenotype based on the genetic risk converted to the relative value, and determining an effective size corresponding to the obtained state as the phenotypic genetic risk.

The obtaining of the phenotypic genetic risk may be performed by obtaining a genetic risk for each of the plurality of phenotypes and obtaining a phenotypic genetic risk based on the obtained plurality of genetic risks, when there is a plurality of phenotypes associated with the disease occurrence.

Yet another aspect of the present invention provides a computer program which is stored in a computer readable recording medium to execute any one method for predicting the risk of the disease combined with the genetic risk for the associated phenotype in a computer.

[Advantageous Effects]

According to the device and the method for predicting the risk for the disease combined with the genetic risk for the associated phenotype of the present invention, a comprehensive genetic risk for the disease is obtained based on the genetic risk for the disease and the genetic risk for the phenotype associated with the disease occurrence, and thus, more objective and accurate disease prediction is possible.

[Description of Drawings]

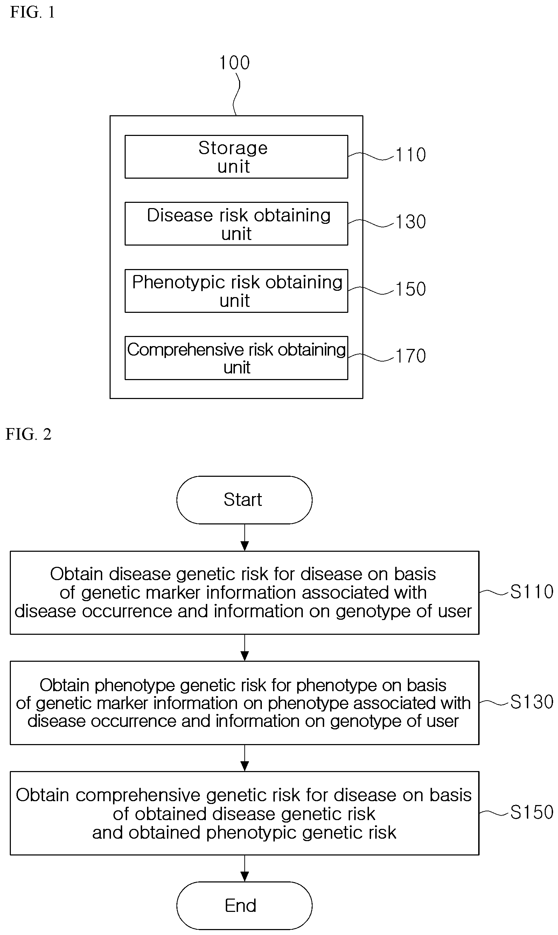

FIG. 1 is a block diagram for describing a device for predicting the risk of a disease combined with the genetic risk of an associated phenotype according to a preferred exemplary embodiment of the present invention. FIG. 2 is a flowchart for describing a method for predicting the risk of a disease combined with the genetic risk of an associated phenotype according to a preferred exemplary embodiment of the present invention.

[Modes of the Invention]

Hereinafter, preferred exemplary embodiments of a device and a method for predicting the risk for a disease combined with the genetic risk for an associated phenotype according to the present invention will be described in detail with reference to the accompanying drawings.

First, a device for predicting the risk of a disease combined with the genetic risk of an associated phenotype according to a preferred exemplary embodiment of the present invention will be described with reference to FIG. 1.

FIG. 1 is a block diagram for describing a device for predicting the risk of a disease combined with the genetic risk of an associated phenotype according to a preferred exemplary embodiment of the present invention.

Referring to FIG. 1, a device (hereinafter, referred to as a 'disease risk prediction device') 100 for predicting the risk for a disease combined with the genetic risk for an associated phenotype according to the preferred exemplary embodiment of the present invention obtains a disease genetic risk for a disease and a phenotypic genetic risk for a phenotype (related diseases, lifestyle, etc.) associated with the disease based on information on a genotype of a user, and obtains a comprehensive genetic risk for the disease based on the disease genetic risk and the phenotypic genetic risk.

To this end, the disease risk prediction device 100 may include a storage unit 110, a disease risk obtaining unit 130, a phenotypic risk obtaining unit 150, and a comprehensive risk obtaining unit 170.

The storage unit 110 serves to store programs and data required for the operation of the disease risk prediction device 100, and may be divided into a program area and a data area.

The program area may store a program for controlling the overall operation of the disease risk prediction device 100, an operating system (OS) for booting the disease risk prediction device 100, an application program required for the operation of the disease risk prediction device 100 such as obtainment of the disease genetic risk, obtainment of the phenotypic genetic risk, and obtainment of the comprehensive genetic risk, and the like.

The data area is an area in which data generated according to the use of the disease risk prediction device 100 is stored, and may store genetic marker information associated with the disease occurrence, information on a phenotype associated with the disease occurrence, genetic marker information on the phenotype, information on an effective size by state of the phenotype, genetic risk information (disease genetic risk, phenotypic genetic risk, comprehensive genetic risk, etc.) of a user for the disease, personal information of the user, and the like.

The disease risk obtaining unit 130 obtains the disease genetic risk for the disease based on the genetic marker information associated with the disease occurrence and the information on the genotype of the user.

Here, the genetic marker information associated with the disease occurrence consists of genetic markers associated with the disease occurrence and is divided for each disease and stored in the storage unit 110. For example, when the disease is "liver cancer", genetic marker information associated with the occurrence of the liver cancer is shown in Table 1.

[Table 1]

| Disease | Genetic marker | Risk factor |
|---|---|---|
| Liver cancer | rs2596542 | T |
| | rs7574865 | G |
| | rs9275319 | A |
| | rs17401966 | A |
| | rs4678680 | G |

For example, the disease risk obtaining unit 130 may obtain the disease genetic risk as shown in Table 2 below based on the genetic marker information associated with the occurrence of the disease (liver cancer) shown in [Table 1] above and the information on the genotype of the user.

[Table 2]

| Disease | Genetic marker | Risk factor | Genotype of user | Disease genetic risk |
|---|---|---|---|---|
| Liver cancer | rs2596542 | T | CT | 5 (= 1+2+0+1+1) |
| | s7574865 | G | GG | |
| | s9275319 | A | GG | |
| | s17401966 | A | AG | |
| | s4678680 | G | GT | |

Here, the disease genetic risk used a genetic risk score method of arithmetically adding the number of risk factors, but the present invention is not limited thereto, and according to an exemplary embodiment, may use various risk calculation methods, such as a genetic risk score, a weighted genetic risk score, a machine learning method, and a linear regression method. In addition, the disease risk obtaining unit 130 may convert the obtained disease genetic risk to a relative value as compared with disease genetic risks of other users in an affiliated group (e.g., the same country, the same residence area, the same age range, etc.) of the user. At this time, the reason for converting the disease genetic risk to the relative value is to normalize (0 to 1) the disease genetic risk to a relative ranking because an absolute value of the genetic risk score may be greatly changed according to the number of genetic markers to be used, and the calculation methods of the disease genetic risk and the phenotypic genetic risk may be different from each other.

For example, the disease risk obtaining unit 130 may convert the obtained disease genetic risk to a percentage (e.g., the top 33%) representing a relative position as compared with the disease genetic risks of other users.

The phenotypic risk obtaining unit 150 obtains the phenotypic genetic risk for the phenotype based on the genetic marker information on the phenotype associated with the disease occurrence and the information on the genotype of the user.

Here, the information on the phenotype associated with the disease occurrence consists of information on a phenotype associated with the disease occurrence, genetic marker information on the phenotype, information on an effective size by state of the phenotype, and the like and is divided for each disease and stored in the storage unit 110. For example, the information on the phenotype related to the occurrence of the liver cancer is shown in [Table 3] below, and the information on the gene marker for the phenotype shown in [Table 3] is as shown in Table 4 to Table 9 below.

[Table 3]

| Disease | Associated phenotype | Number of state (grade) | Effective size by state | | |
|---|---|---|---|---|---|
| Liver cancer | Drinking (alcohol dependence) | Stage 3 | 4.36 times (High) | 1.27 times (Medium) | 1 time (Low) |
| | Smoking (nicotine dependence) | Stage 2 | 1.50 times (smoking) | 1 time (non-smoking) | |
| | Body mass index | Stage 3 | 1.83 times (Obesity) | 1.18 times (Overweight) | 1 time (Normal) |
| | Depression | Stage 3 | 1.20 times (High) | 1 time (Medium) | 1 time (Low) |
| | Non-alcoholic steatohepatitis | Stage 2 | 3.10 times (High) | 1 time (Low) | |
| | Hepatocirrhosis | Stage 2 | 2.55 times (High) | 1 time (Low) | |

[Table 4]

| Phenotype | Genetic marker | Risk factor |
|---|---|---|
| Drinking (alcohol dependence) | rs10741210 | G |
| | rs1793257 | T |
| | rs2400954 | C |
| | rs10483282 | T |
| | rs2154294 | G |

[Table 5]

| Phenotype | Genetic marker | Risk factor |
|---|---|---|
| Smoking (nicotine dependence) | rs4142041 | G |
| | rs6265 | C |
| | rs2036527 | A |
| | rs667282 | C |
| | rs3733829 | G |

[Table 6]

| Phenotype | Genetic marker | Risk factor |
|---|---|---|
| Body mass index | rs17094222 | C |
| | rs4146429 | C |
| | rs7903146 | C |
| | rs718948 | C |
| | rs751008 | A |

[Table 7]

| Phenotype | Genetic marker | Risk factor |
|---|---|---|
| Depression | rs1457614 | C |
| | rs4238010 | G |
| | rs9943849 | T |
| | rs1545843 | A |
| | rs9572423 | G |

[Table 8]

| Phenotype | Genetic marker | Risk factor |
|---|---|---|
| Non-alcoholic steatohepatitis | rs343064 | T |
| | rs1836127 | T |
| | rs643608 | C |
| | rs4243849 | G |
| | rs2358462 | C |

[Table 9]

| Phenotype | Genetic marker | Risk factor |
|---|---|---|
| Hepatocirrhosis | rs738409 | G |
| | rs10401969 | C |
| | rs6441286 | G |
| | rs3790567 | A |
| | rs907092 | A |

At this time, when there is a plurality of phenotypes associated with the disease occurrence, the phenotypic risk obtaining unit 150 may obtain a genetic risk for each of the plurality of phenotypes and obtain phenotypic genetic risks based on the obtained plurality of genetic risks. For example, when the disease is liver cancer, as shown in [Table 3], since the number of phenotypes associated with the occurrence of the liver cancer is six, the phenotypic risk obtaining unit 150 may obtain a genetic risk for each of the six phenotypes and obtain phenotypic genetic risks based on the obtained six genetic risks. In addition, the phenotypic risk obtaining unit 150 may convert the obtained phenotypic genetic risk to a relative value as compared with phenotypic genetic risks of other users in an affiliated group (e.g., the same country, the same residence area, the same age range, etc.) of the user. At this time, the reason for converting the phenotypic genetic risk to the relative value is to normalize (0 to 1) the phenotypic genetic risk to a relative ranking because an absolute value of the genetic risk score may be greatly changed according to the number of genetic markers to be used, and the calculation methods of the disease genetic risk and the phenotypic genetic risk may be different from each other. Here, when there is a plurality of phenotypes associated with the disease occurrence, the phenotypic risk obtaining unit 150 may convert the genetic risks obtained for the plurality of phenotypes to relative values as compared with the corresponding genetic risks of other users, respectively, and obtain phenotypic genetic risks based on the plurality of genetic risks converted to the relative values.

More specifically, the phenotypic risk obtaining unit 150 may obtain the phenotypic genetic risk based on the genetic marker information on the phenotype and the information on the genotype of the user by using a predefined effective size by state of the phenotype.

That is, the phenotypic risk obtaining unit 150 may obtain a genetic risk based on the genetic marker information on the phenotype and the information on the genotype of the user, convert the obtained genetic risk to the relative value as compared with the genetic risks for the phenotypes of other users in the affiliated group of the user, obtain the state of the phenotype based on the genetic risk converted to the relative value, and determine an effective size corresponding to the obtained state as the phenotypic genetic risk.

For example, when the disease is liver cancer, the phenotypic risk obtaining unit 150 may obtain the phenotypic genetic risk as shown in Table 10 below based on the information on the genotype of the user and Table 3 to Table 9 above.

[Table 10]

| Phenotype | Genetic marker | Risk factor | Genotype of user | Phenotypic genetic risk | Relative state (user grade to group) | Effective size of user according to relative state | Phenotypic genetic risk |
|---|---|---|---|---|---|---|---|
| Drinking (alcohol dependence) | rs10741210 | G | AG | 7 | Top 8% (high) | 4.36 | 11.64 (= 4.36 + 1 + 1.18 + 1 + 3.10 + 1) (When convented to relative value top 7%) |
| | rs1793257 | T | CT | | | | |
| | rs2400954 | C | CC | | | | |
| | rs10483282 | T | TT | | | | |
| | rs2154294 | G | AG | | | | |
| Smoking (nicotine dependence) | rs4142041 | G | AA | 2 | Top 98% (non-smoking) | 1 | |
| | rs6265 | C | TT | | | | |
| | rs2036527 | A | AG | | | | |
| | rs667282 | C | TT | | | | |
| | rs3733829 | G | AG | | | | |
| Body mass index | rs17094222 | C | CC | 6 | Top 39% (overweight) | 1.18 | |
| | rs4146429 | C | CC | | | | |
| | rs7903146 | C | TT | | | | |
| | rs718948 | C | TT | | | | |
| | rs751008 | A | AA | | | | |
| Dpression | rs1457614 | C | AA | 0 | Top 100% (low) | 1 | |
| | rs4238010 | G | AA | | | | |
| | rs9943849 | T | CC | | | | |
| | rs1545843 | A | GG | | | | |
| | rs9572423 | G | AA | | | | |
| Non-alcoholic steatohepatitis | rs343064 | T | CT | 7 | Top 5% (high) | 3.10 | |
| | rs1836127 | T | GT | | | | |
| | rs643608 | C | CC | | | | |
| | rs4243849 | G | AG | | | | |
| | rs2358462 | C | CC | | | | |
| Hepato cirrhosis | rs738409 | G | CC | 3 | Top 71% (low) | 1 | |
| | rs10401969 | C | CT | | | | |
| | rs6441286 | G | TT | | | | |
| | rs3790567 | A | AG | | | | |
| | rs907092 | A | AG | | | | |

Here, the phenotypic genetic risk used a genetic risk score method of arithmetically adding the number of risk factors, but the present invention is not limited thereto, and according to an exemplary embodiment, may use various risk calculation methods such as a genetic risk score, a weighted genetic risk score, a machine learning method, a linear regression method, and the like.

The comprehensive risk obtaining unit 170 may obtain a comprehensive genetic risk for the disease based on the disease genetic risk obtained by the disease risk obtaining unit 130 and the phenotypic genetic risk obtained by the phenotypic risk obtaining unit 150.

At this time, the comprehensive risk obtaining unit 170 may obtain the comprehensive genetic risk based on the disease genetic risk converted to the relative value and the phenotypic genetic risk converted to the relative value.

More specifically, the comprehensive risk obtaining unit 170 may obtain the comprehensive genetic risk based on the disease genetic risk and the phenotypic genetic risk by using a ratio of genetic factors affecting the disease occurrence.

Here, the ratio of genetic factors affecting the disease occurrence represents a proportion at which the genetic factors affect the occurrence of a specific disease. For example, when the disease is "liver cancer" and the proportion of the genetic factors affecting the occurrence of the liver cancer is "61%", the ratio of the genetic factors may be "0.61."

For example, the comprehensive risk obtaining unit 170 may obtain the comprehensive genetic risk for the disease through the following Equation 1.

[Equation 1]

$$\text{Comprehensive genetic risk} = a * \text{disease genetic risk} + (1\text{-}a) * \text{phenotypic genetic risk}$$

Here, a represents a ratio of genetic factors that affect the disease occurrence. The disease genetic risk and the phenotypic genetic risk may also use values converted to the relative values.

For example, when the disease is "liver cancer", the disease genetic risk converted to the relative value is "the top 33%." and the phenotypic genetic risk converted to the relative value is "the top 7%", the comprehensive genetic risk for the liver cancer is as shown in Equation 2 below.

[Equation 2]

$$0.61 * 0.33 + (1 - 0.61) * 0.07 = 0.23$$

In addition, the comprehensive risk obtaining unit 170 may convert the obtained comprehensive genetic risk to a relative value as compared with comprehensive genetic risks of other users in the affiliated group of the user.

For example, the comprehensive risk obtaining unit 170 may obtain the obtained comprehensive genetic risk as a relative ranking as compared with the comprehensive genetic risks of other users and rank the disease risk as follows based on the obtained relative ranking.

Top 1% to 25%: Disease risk grade "high"
Top 26% to 50%: Disease risk grade "mid-high"
Top 51% to 75%: Disease risk grade "mid-low"
Top 76% to 100%: Disease risk grade "low"

Then, a method for predicting the risk of a disease combined with the genetic risk of an associated phenotype according to a preferred exemplary embodiment of the present invention will be described with reference to FIG. 2.

FIG. 2 is a flowchart for describing a method for predicting the risk of a disease combined with the genetic risk of an associated phenotype according to a preferred exemplary embodiment of the present invention.

Referring to FIG. 2, the disease risk prediction device 100 obtains a disease genetic risk for a disease based on genetic marker information associated with the disease occurrence and information on a genotype of a user (S110). At this time, the disease risk prediction device 100 may convert the obtained disease genetic risk to a relative value as compared with disease genetic risks of other users in an affiliated group (e.g., the same country, the same residence area, the same age range, etc.) of the user.

Next, the disease risk prediction device 100 obtains a phenotypic genetic risk for a phenotype based on genetic marker information on a phenotype associated with the disease occurrence and the information on the genotype of the user (S 130).

At this time, when there is a plurality of phenotypes associated with the disease occurrence, the disease risk prediction device 100 may obtain a genetic risk for each of the plurality of phenotypes and obtain phenotypic genetic risks based on the obtained plurality of genetic risks.

In addition, the disease risk prediction device 100 may convert the obtained phenotypic genetic risk to a relative value as compared with phenotypic genetic risks of other users in an affiliated group (e.g., the same country, the same residence area, the same age range, etc.) of the user. Here, when there is a plurality of phenotypes related to the disease occurrence, the disease risk prediction device 100 may convert the genetic risks obtained for the plurality of phenotypes to relative values as compared with the corresponding genetic risks of other users, respectively, and obtain the phenotypic genetic risks based on the plurality of genetic risks converted to the relative values.

More specifically, the disease risk prediction device 100 may obtain the phenotypic genetic risk based on the genetic marker information on the phenotype and the information on the genotype of the user by using a predefined effective size by state of the phenotype. That is, the disease risk prediction device 100 may obtain a genetic risk based on the genetic marker information on the phenotype and the information on the genotype of the user, convert the obtained genetic risk to the relative value as compared with the genetic risks for the phenotypes of other users in the affiliated group of the user, obtain the state of the phenotype based on the genetic risk converted to the relative value, and determine an effective size corresponding to the obtained state as the phenotypic genetic risk.

Thereafter, the disease risk prediction device 100 may obtain a comprehensive genetic risk for the disease based on the obtained disease genetic risk and the obtained phenotypic genetic risk (S150).

At this time, the disease risk prediction device 100 may obtain the comprehensive genetic risk based on the disease genetic risk converted to the relative value and the phenotypic genetic risk converted to the relative value.

More specifically, the disease risk prediction device 100 may obtain the comprehensive genetic risk based on the disease genetic risk and the phenotypic genetic risk by using a ratio of genetic factors affecting the disease occurrence.

In addition, the disease risk prediction device 100 may convert the obtained comprehensive genetic risk to a relative value as compared with comprehensive genetic risks of other users in the affiliated group of the user.

The present invention can be implemented as computer readable codes in a computer readable recording medium. The computer readable recording medium includes all kinds of recording devices for storing data which may be read by a computer. Examples of the computer readable recording medium include a ROM, a RAM, a CD-ROM, a magnetic tape, a floppy disk, an optical data storage device, and the like.

While the preferred exemplary embodiments of the present invention have been illustrated and described above, the present invention is not limited to the aforementioned specific preferred exemplary embodiments, various modifications may be made by a person with ordinary skill in the technical field to which the present invention pertains without departing from the subject matters of the present invention that are claimed in the claims, and these modifications are included in the scope of the claims.

[Explanation of Reference Numerals and Symbols]

100:     Disease risk prediction device
110:     Storage unit
130:     Disease risk obtaining unit
150:     Phenotypic risk obtaining unit
170:     Comprehensive risk obtaining unit

**Claims**

1. A device for predicting a risk of a disease combined with a genetic risk for an associated phenotype, the device comprising:

   a disease risk obtaining unit configured to obtain a disease genetic risk for the disease based on genetic marker information associated with the disease occurrence and information on a genotype of a user;
   a phenotypic risk obtaining unit configured to obtain a phenotypic genetic risk for a phenotype based on the genetic marker information on the phenotype associated with the disease occurrence and the information on the genotype of the user; and
   a comprehensive risk obtaining unit configured to obtain a comprehensive genetic risk for the disease based on the disease genetic risk obtained by the disease risk obtaining unit and the phenotypic genetic risk obtained by the phenotypic risk obtaining unit.

2. The device of claim 1, wherein the comprehensive risk obtaining unit obtains the comprehensive genetic risk based on the disease genetic risk and the phenotypic genetic risk by using a ratio of genetic factors affecting the disease occurrence.

3. The device of claim 1, wherein the disease risk obtaining unit converts the obtained disease genetic risk to a relative value as compared with disease genetic risks of other users in an affiliated group of the user,

the phenotypic risk obtaining unit converts the obtained phenotypic genetic risk to a relative value as compared with phenotypic genetic risks of other users in the affiliated group of the user, and
the comprehensive risk obtaining unit obtains the comprehensive genetic risk based on the disease genetic risk converted to the relative value and the phenotypic genetic risk converted to the relative value and converts the obtained comprehensive genetic risk to a relative value as compared with comprehensive genetic risks of other users in the affiliated group of the user.

4. The device of claim 1, wherein the phenotypic risk obtaining unit obtains the phenotypic genetic risk based on the genetic marker information on the phenotype and the information on the genotype of the user by using a predefined effective size by state of the phenotype.

5. The device of claim 4, wherein the phenotypic risk obtaining unit obtains a genetic risk based on the genetic marker information on the phenotype and the information on the genotype of the user, converts the obtained genetic risk to a relative value as compared with the genetic risks for the phenotypes of other users in an affiliated group of the user, obtains the state of the phenotype based on the genetic risk converted to the relative value, and determines an effective size corresponding to the obtained state as the phenotypic genetic risk.

6. The device of claim 1, wherein when there is the plurality of phenotypes associated with the disease occurrence, the phenotypic risk obtaining unit obtains a genetic risk for each of the plurality of phenotypes and obtains the phenotypic genetic risk based on the obtained plurality of genetic risks.

7. A method for predicting a risk of a disease combined with a genetic risk for an associated phenotype, the method comprising:

obtaining a disease genetic risk for the disease based on genetic marker information associated with the disease occurrence and information on a genotype of a user;
obtaining a phenotypic genetic risk for the phenotype based on the genetic marker information on the phenotype associated with the disease occurrence and the information on the genotype of the user; and
obtaining a comprehensive genetic risk for the disease based on the obtained disease genetic risk and the obtained phenotypic genetic risk.

8. The method of claim 7, wherein the obtaining of the comprehensive genetic risk is performed by obtaining the comprehensive genetic risk based on the disease genetic risk and the phenotypic genetic risk by using a ratio of genetic factors affecting the disease occurrence.

9. The method of claim 7, wherein the obtaining of the disease genetic risk is performed by converting the obtained disease genetic risk to a relative value as compared with disease genetic risks of other users in an affiliated group of the user,

the obtaining of the phenotypic genetic risk is performed by converting the obtained phenotypic genetic risk to a relative value as compared with phenotypic genetic risks of other users in the affiliated group of the user, and
the obtaining of the comprehensive genetic risk is performed by obtaining the comprehensive genetic risk based on the disease genetic risk converted to the relative value and the phenotypic genetic risk converted to the relative value and converting the obtained comprehensive genetic risk to a relative value as compared with comprehensive genetic risks of other users in the affiliated group of the user.

10. The method of claim 7, wherein the obtaining of the phenotypic genetic risk is performed by obtaining the phenotypic genetic risk based on the genetic marker information on the phenotype and the information on the genotype of the user by using a predefined effective size by state of the phenotype.

11. The method of claim 10, wherein the obtaining of the phenotypic genetic risk is performed by obtaining a genetic risk based on the genetic marker information on the phenotype and the information on the genotype of the user, converting the obtained genetic risk to a relative value as compared with the genetic risks for the phenotypes of other users in the affiliated group of the user, obtaining the state of the phenotype based on the genetic risk converted to the relative value, and determining an effective size corresponding to the obtained state as the phenotypic genetic risk.

**12.** The method of claim 7, wherein the obtaining of the phenotypic genetic risk is performed by obtaining a genetic risk for each of the plurality of phenotypes and obtaining a phenotypic genetic risk based on the obtained plurality of genetic risks, when there is the plurality of phenotypes associated with the disease occurrence.

**13.** A computer program stored in a computer readable recording medium to execute the method for predicting the risk of the disease combined with the genetic risk for the associated phenotype of any one of claims 7 to 12 in a computer.

FIG. 1

100

```
┌─────────────────────────────────────┐
│  ┌───────────────────────────────┐   │
│  │          Storage              │   │~110
│  │           unit                │   │
│  └───────────────────────────────┘   │
│  ┌───────────────────────────────┐   │
│  │    Disease risk obtaining     │   │~130
│  │            unit               │   │
│  └───────────────────────────────┘   │
│  ┌───────────────────────────────┐   │
│  │   Phenotypic risk obtaining   │   │~150
│  │            unit               │   │
│  └───────────────────────────────┘   │
│  ┌───────────────────────────────┐   │
│  │  Comprehensive risk obtaining │   │~170
│  │            unit               │   │
│  └───────────────────────────────┘   │
└─────────────────────────────────────┘
```

FIG. 2

Start

Obtain disease genetic risk for disease on basis
of genetic marker information associated with
disease occurrence and information on genotype of user ~S110

Obtain phenotype genetic risk for phenotype on basis
of genetic marker information on phenotype associated with
disease occurrence and information on genotype of user ~S130

Obtain comprehensive genetic risk for disease on basis
of obtained disease genetic risk
and obtained phenotypic genetic risk ~S150

End

**INTERNATIONAL SEARCH REPORT**

| International application No. |
| --- |
| **PCT/KR2019/010258** |

### A. CLASSIFICATION OF SUBJECT MATTER

*G16H 50/30(2018.01)i, G16H 50/20(2018.01)i, A61B 5/00(2006.01)i*

According to International Patent Classification (IPC) or to both national classification and IPC

### B. FIELDS SEARCHED

Minimum documentation searched (classification system followed by classification symbols)
G16H 50/30; A61B 5/1455; G01N 33/48; G06F 19/18; G06F 19/20; G16B 20/00; G16B 5/00; G16B 50/00; G16H 50/20; A61B 5/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: disease, gene, marker, phenotype

### C. DOCUMENTS CONSIDERED TO BE RELEVANT

| Category* | Citation of document, with indication, where appropriate, of the relevant passages | Relevant to claim No. |
| --- | --- | --- |
| Y | KR 10-2019-0077997 A (CLINOMICS CO.) 04 July 2019<br>See paragraphs [0038]-[0088], claims 1-10, and figures 1-2. | 1-13 |
| Y | KR 10-1693510 B1 (SYNTEKABIO CO., LTD.) 17 January 2017<br>See paragraphs [0049]-[0088], claim 1, and figures 2-5. | 1-13 |
| Y | KR 10-2017-0134203 A (MEDIZEN HUMANCARE INC.) 06 December 2017<br>See paragraphs [0047]-[0057], claims 1-7, and figures 4-5. | 1-13 |
| Y | JP 2016-048251 A (SONY CORP.) 07 April 2016<br>See paragraphs [0070]-[0076]. | 1-13 |
| A | KR 10-1693504 B1 (SYNTEKABIO CO., LTD.) 17 January 2017<br>See paragraphs [0047]-[0077], and figures 2-6. | 1-13 |

| ☐ Further documents are listed in the continuation of Box C. | ☒ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 08 MAY 2020 (08.05.2020) | **08 MAY 2020 (08.05.2020)** |

| Name and mailing address of the ISA/KR<br>Korean Intellectual Property Office<br>Government Complex Daejeon Building 4, 189, Cheongsa-ro, Seo-gu,<br>Daejeon, 35208, Republic of Korea<br>Facsimile No. +82-42-481-8578 | Authorized officer<br><br>Telephone No. |
| --- | --- |

Form PCT/ISA/210 (second sheet) (January 2015)

INTERNATIONAL SEARCH REPORT
Information on patent family members

International application No.

**PCT/KR2019/010258**

| Patent document cited in search report | Publication date | Patent family member | Publication date |
|---|---|---|---|
| KR 10-2019-0077997 A | 04/07/2019 | KR 10-2097540 B1 | 07/04/2020 |
| KR 10-1693510 B1 | 17/01/2017 | US 2019-0087540 A1 | 21/03/2019 |
| | | WO 2017-116135 A1 | 06/07/2017 |
| KR 10-2017-0134203 A | 06/12/2017 | CN 109196590 A | 11/01/2019 |
| | | KR 10-2019-0080832 A | 08/07/2019 |
| | | WO 2017-204482 A2 | 30/11/2017 |
| | | WO 2017-204482 A3 | 18/01/2018 |
| JP 2016-048251 A | 07/04/2016 | AU 2011-275061 A1 | 10/01/2013 |
| | | AU 2011-275061 B2 | 25/09/2014 |
| | | CA 2800461 A1 | 12/01/2012 |
| | | CN 102959081 A | 06/03/2013 |
| | | EP 2592142 A1 | 15/05/2013 |
| | | JP 2012-030038 A | 16/02/2012 |
| | | JP 2018-042560 A | 22/03/2018 |
| | | JP 5838557 B2 | 06/01/2016 |
| | | JP 6222202 B2 | 01/11/2017 |
| | | JP 6620951 B2 | 18/12/2019 |
| | | KR 10-1919623 B1 | 16/11/2018 |
| | | KR 10-2013-0094730 A | 26/08/2013 |
| | | MX 2012014926 A | 20/05/2013 |
| | | MX 338056 B | 01/04/2016 |
| | | SG 186352 A1 | 30/01/2013 |
| | | TW 201229802 A | 16/07/2012 |
| | | TW I454957 B | 01/10/2014 |
| | | US 10303845 B2 | 28/05/2019 |
| | | US 2013-0110409 A1 | 02/05/2013 |
| | | US 2019-0237164 A1 | 01/08/2019 |
| | | WO 2012-005138 A1 | 12/01/2012 |
| KR 10-1693504 B1 | 17/01/2017 | WO 2017-116123 A1 | 06/07/2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20190077997

**Non-patent literature cited in the description**

- **SCHRODI SJ et al.** *Front. Genet.,* 2014

- **GOLDSTEIN BA et al.** *Front. Genet.,* 2014